# EUROPEAN PATENT APPLICATION

(11) **EP 2 368 874 A1**
(43) Date of publication of application: **28.09.2011**
(21) Application number: 10157913.4
(22) Date of filing: 26.03.2010
(51) Int. Cl.: C07C 269/06, C07C 271/22

(54) **Racemisation of (R)-N-Boc-3-hydroxyadamant-1-yl glycine**

(71) Applicant: Sandoz AG, 4056 Basel (CH)
(72) Inventor: The designation of the inventor has not yet been filed
(74) Representative: Wichmann, Hendrik

(57) **Abstract**

The present invention refers to the preparation of racemic N-Boc-3-hydroxyadamant-1-yl glycine.

## Description

The present invention refers to the preparation of racemic N-Boc-3-hydroxyadamant-1-yl glycine from compositions containing (R)-N-Boc-3-hydroxyadamant-1-yl glycine and optionally the use of racemic N-Boc-3-hydroxyadamant-1-yl glycine for the preparation of (S)-N-Boc-3-hydroxyadamant-1-yl glycine. For the purposes of this invention the abbreviation "Boc" means Di-tert-butyldicarbonate.

(S)-N-Boc-3-hydroxyadamant-1-yl glycine is a key intermediate of therapeutic compounds which act as dipeptidyl peptidase IV inhibitors including Saxagliptin.

Saxagliptin (1S,3S,SS)-2-[(2S)-2-amino-2-(3-hydroxy-1-adamantyl)acetyl]-2-azabicyclo [3.1.0]hexane-3-carbonitrile or its hydrochloride salt is an orally active reversible dipeptidyl peptidase-4 (DD4) inhibitor, which is a therapeutic agent for treatment of type-2 diabetes mellitus, obesity or related diseases, and is disclosed for example in US 6,395,767 B2, example 60.

Saxagliptin can be produced by coupling (S)-N-Boc-3-hydroxyadamant-1-yl glycine and methanoprolineamide as shown in the following scheme:

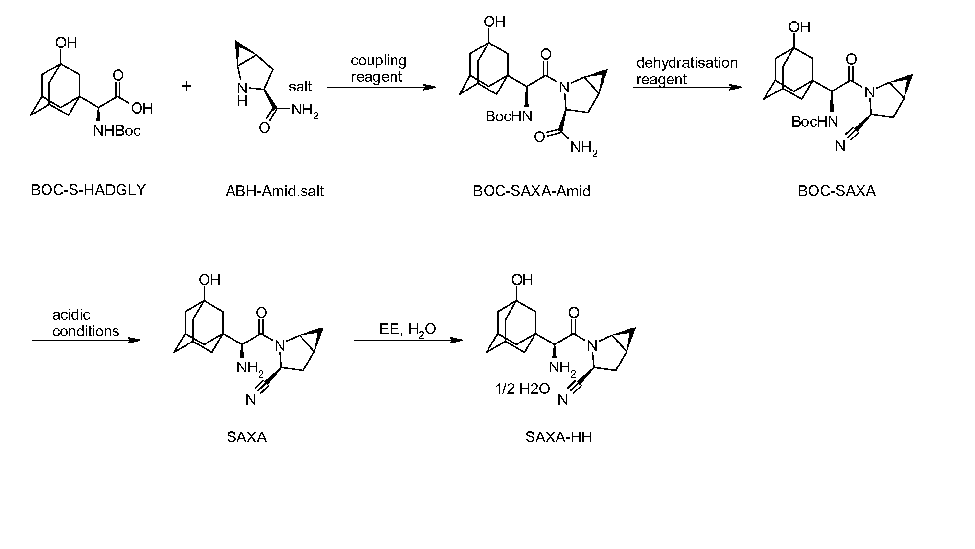

The prolineamide moiety is subsequently dehydrated to give the cyanide. Removal of the Boc protecting group, followed by neutralization gives Saxagliptin. N-Boc-3-hydroxyadamant-1-yl glycine comprises two enantiomers, i. e. *(R)*-N-Boc-3-hydroxyadamant-1-yl glycine and *(S)*-N-Boc-3-hydroxyadamant-1-yl glycine. For the synthesis of Saxagliptin *(S)*-N-Boc-3-hydroxyadamant-1-yl glycine has to be used.

According to WO 2004/052850 A2 *(S)*-N-Boc-3-hydroxyadamant-1-yl glycine can be prepared by enzymatic reductive amination using e. g. an extract from recombinant escherichia coli which is a rather complex reaction or by synthesis of racemic N-Boc-3-hydroxyadamant-1-yl glycine starting from 3-hydroxyadamant-1-yl-bromoacetic acid following a racemic cleavage with a chiral base such as ([1R, 2S]-(-)-1,2-diphenylhydroxy ethylamine. The disadvantage of this process is that half of the amino acid is lost because (R)-N-Boc-3-hydroxyadamant-1-yl glycine cannot be used.

Though it is known from e. g. L. Chen et al., J. Org. Chem. 2006, 71, 5468 to recycle undesired enantiomer by converting the undesired enantiomer from e. g. the supernatant of the desired enantiomer by exposure to diazabicyclooctane and sequent hydrolysis there is no suggestion in the prior art how to recycle (R)-N-Boc-3-hydroxyadamant-1-yl glycine and to transform it into (S)-N-Boc-3-hydroxyadamant-1-yl glycine for the synthesis of Saxagliptin.

It was an object of the present invention to avoid the loss of amino acid described supra and to optimize the preparation for (S)-N-Boc-3-hydroxyadamant-1-yl glycine.

In one embodiment the present invention refers to a process for the preparation of racemic N-Boc-3-hydroxyadamant-1-yl glycine, wherein the educt (R)-N-Boc-3-hydroxyadamant-1-yl glycine or an isolated or not isolated derivative thereof is treated with a base under racemisation to yield racemic N-Boc-3-hydroxyadamant-1-yl glycine or the racemic derivative thereof and hydrolysis of the racemic N-Boc-3-hydroxyadamant-1-yl glycine derivative to yield N-Boc-3-hydroxyadamant-1-yl glycine and optionally reacting or transforming the racemic N-Boc-3-hydroxyadamant-1-yl glycine in further process steps.

For illustrative purposes a possible reaction scheme using carbonyldiimidazol (CDI) as an activator and DMSO as solvent is shown as follows:

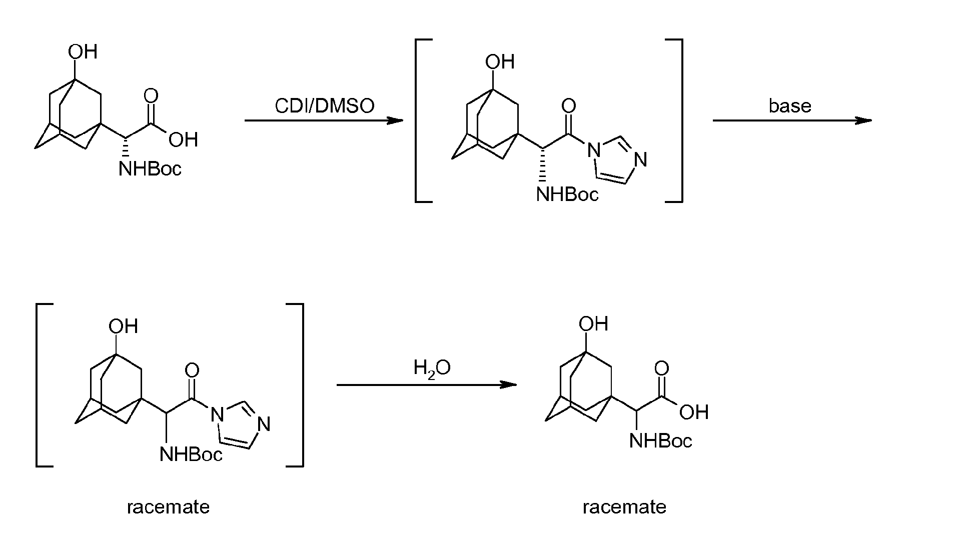

According to the present invention it is possible to transfer (R)-N-Boc-3-hydroxyadamant-1-yl glycine into the racemic compound and to gain the desired (S)-N-Boc-3-hydroxyadamant-1-yl glycine and to isolate it.

In further embodiments the present invention refers to a process for the preparation of *(S)-*N-Boc-3-hydroxyadamant-1-yl glycine wherein the educt (R)-N-Boc-3-hydroxyadamant-1-yl glycine or an isolated or not isolated derivative thereof is treated with a base under racemisation to yield racemic N-Boc-3-hydroxyadamant-1-yl glycine or the racemic derivative thereof and hydrolysis of the racemic N-Boc-3-hydroxyadamant-1-yl glycine derivative to yield N-Boc-3-hydroxyadamant-1-yl glycine and removing *(R)*-N-Boc-3-hydroxyadamant-1-yl glycine and/or isolating (S)-N-Boc-3-hydroxyadamant-1-yl glycine.

In a further embodiment of the present invention the (S)-N-Boc-3-hydroxyadamant-1-yl glycine enriched composition obtained according to the present invention is used for the preparation of Saxagliptin.

According to the present invention the educt, i. e. (R)-N-Boc-3-hydroxyadamant-1-yl glycine may be present in a mixture with (S)-N-Boc-3-hydroxyadamant-1-yl glycine or as a solution containing essentially (R)-N-Boc-3-hydroxyadamant-1-yl glycine. In a preferred embodiment, the educt (R)-N-Boc-3-hydroxyadamant-1-yl glycine is used in a purity of from 20 to 99.9%, based on the weight of the educt used.

The racemisation is effected by a base which may be used in catalytic amounts, preferably in an amount of 0.1 to 0.5 % based on the weight of the educt used, or the base may be used in an amount of 1 to 6 equivalents, preferably in an essentially equivalent molar amount based on the educt used.

Preferred bases include inorganic hydrides, preferably NaH, metal organic compounds, preferably butyl lithium, organic amines, preferably tertiary amines, e. g. diazabicyclooctane (DABCO) and trimethyl amine (TEA), inorganic carbonates such as Na₂C0₃, K₂CO₃.

Preferably the racemisation is conducted at a pH of from 9 to 25, preferably at a pH of from 12 to 17. It is preferred to adjust the pH value with the base used in the process.

The racemisation is preferably conducted at a temperature of from -30 to 80 °C, especially at a temperature of from 0 to 30 °C.

It is possible to conduct the racemisation in the absence of an activation agent or in the presence of an activation agent. In a preferred embodiment *(R)*-N-Boc-3-hydroxyadamant-1-yl glycine is reacted with an activation reagent prior to racemisation. Preferred activation agents increase the acidity of the H atom in α-position to the carbonyl-group of the glycine. Preferred activation agents include carbodiimides such as dicyclohexylcarbodiimid (DCC), N-(3-dimethylaminopropyl-N'-ethylcarbodiimid (EDC), inorganic acid chlorides such as SOCl₂, chloroformic acid esters such as ClCO₂-i-butyl, fluorinated phenols such as pentafluorophenol, sulfonic acid chlorides including mesitylchloride and tosylfluoride and carbonyl diheteroaromatic compounds such as carbonyldiimidazole (CDI).

Preferred solvents for the racemisation are non-protic polar solvents, preferably solvents with a dielectricity constant greater than 15, especially dimethylsulfoxide (DMSO) acetonitrile, dimethylformamide (DMF), acetone, dimethylether (DME), dioxane, dimethylpropylene urea (DMPU) and mixtures thereof.

In a preferred embodiment the racemic N-Boc-3-hydroxyadamant-1-yl glycine obtained is subjected to a separation into its enantiomers. In a further embodiment of the present invention the racemic solution of N-Boc-3-hydroxyadamant-1-yl glycine obtained from *(R)*-N-Boc-3-hydroxyadamant-1-yl glycine is reacted with a chiral base to crystallize *(S)-*N-Boc-3-hydroxyadamant-1-yl glycine and to obtain it in enantiomerically pure form. Suitable chiral bases is e. g. (1R,2S)-1,2-diphenyl-2-hydroxyethyl amine and S-(-)-1-1(1-naphthyl)ethylamine.

In a further embodiment of the present invention the *(S)*-N-Boc-3-hydroxyadamant-1-yl glycine obtained via racemic N-Boc-3-hydroxyadamant-1-yl glycine from *(R)*-N-Boc-3-hydroxyadamant-1-yl glycine is used for the synthesis of Saxagliptin.

### Examples

### Example 1

Racemisation using dimethyl sulfoxide (DMSO) as solvent, sodium hydrate as base and CDI as activator

To a solution of 1g (R)-N-Boc-3-hydroxyadamant-1-yl glycine (3.07mmol, leq, 74.4%ee) in 20mL DMSO was added 0.55g Carbonyldiimidazol (3.4mmol, 1.1 eq) at 20-25°C and stirred for 4.5h. conversion was followed by HPLC. Afterwards 0.16g sodium hydride (60% in Oleum, 4.5mmol, 1.5eq) was added and the mixture was stirred for 4h at 50°C turning into a yellow solution. Afterwards the mixture was stirred for 48h at 20-25°C. Afterwards the mixture was hydrolysed by adding 152L 1M NaOH solution and stirring for 1.5h. To the mixture 20mL water and 40mL methyl tertiary butyl ether (MTBE) was added and pH 3 was adjusted by adding 5M HCl. After phase separation the organic phase was washed with 20mL water and 20mL saturated NaCl solution, dried over sodium sulfate, filtered and after removal of the organic solvent dried under vacuum. 0.7g of a white amorphous solid identified as racemic N-Boc-3-hydroxyadamant-1-yl glycine was isolated (70%, 2.15mmo1, 15.4%ee).

### Example 2

### Alternative Procedure: Without heating

To a solution of 1g (R)-N-Boc-3-hydroxyadamant-1-yl glycine (3.07mmol, leq, 74.4%ee) in 20mL DMSO was added 0.55g Carbonyldiimidazol (3.4mmol, 1.1 eq) at 20-25°C and stirred for 4.5h. conversion was followed by HPLC. Afterwards 0.18g sodium hydride (60% in oleum, 4.5mmol, 1.5eq) was added and the mixture was stirred 38h at 20-25°C turning into a slightly yellow solution. Afterwards the mixture was hydrolyzed by adding 152mL 1M NaOH solution and stirring for 40min. To the mixture 20mL water and 40mL MTBE was added and pH 3 was adjusted by adding 5M HCl. After phase separation the organic phase was washed with 30mL water and 30mL saturated NaCl solution, dried over sodium sulfate, filtered and after removal of the organic solvent dried under vacuum. 0.75g of a white amorphous solid identified as racemic N-Boc-3-hydroxyadamant-1-yl glycine was isolated (75%, 2.30mmo1, 14.4%ee).

### Example 3

### Alternative Reaction Procedure: Potassium carbonate as base

To a solution of 1g (R)-N-Boc-3-hydroxyadamant-1-yl glycine (3.07mmol, leq, 76.3%ee) in 20mL DMSO was added 0.55g Carbonyldiimidazol (3.4mmol, 1.1 eq) at 20-25°C and stirred for 4.5h. conversion was followed by HPLC. Afterwards 0.64g potassium carbonate (4.5mmol, 1.5eq) was added and the mixture was stirred for 2.5h at 50°C turning into a yellow suspension. Afterwards the mixture was stirred for 39h at 20-25°C. Afterwards the mixture was hydrolysed by adding 152L 1M NaOH solution and stirring for 1.5h. To the mixture 20mL water and 40mL MTBE was added and pH 3 was adjusted by adding 5M HCl. After phase separation the organic phase was washed with 30mL water and 30mL saturated NaCl solution, dried over sodium sulfate, filtered and after removal of the organic solvent dried under vacuum. 0.6g of a white amorphous solid identified as racemic N-Boc-3-hydroxyadamant-1-yl glycine was isolated (60%, 1.84mmol, 12.3%ee).

### Example 4:

### Alternative reaction procedure: 3 eq potassium carbonate as base

To a solution of 5g (R)-N-Boc-3-hydroxyadamant-1-yl glycine (15.4mmol, leq, 76.3%ee) in 100mL DMSO was added 2.75g Carbonyldiimidazol (17.0mmol, 1.1 eq) at 20-25°C and stirred for 4.5h. conversion was followed by HPLC. Afterwards 6.4g potassium carbonate (46.3mmol, 3.0eq) was added and the mixture was stirred for 24h at 20-25°C. Afterwards the mixture was hydrolysed by adding 100mL 1M NaOH solution followed by 80mL of water. To the mixture 150mL tert-butylmethylether, MTBE, was added and pH 3 was adjusted by adding 2M HCl. After phase separation the organic phase was washed with 100mL saturated NaCl solution, dried over sodium sulfate, filtered and after removal of the organic solvent dried under vacuum. 3.6g of a white amorphous solid identified as racemic N-Boc-3-hydroxyadamant-1-yl glycine was isolated (72%, 11.1mmol, 8.8%ee).

### Example 5

### Alternative reaction procedure: Iso-butyl chloroformiate as activating reagent

To a solution of 1g (R)-N-Boc-3-hydroxyadamant-1-yl glycine (3.07mmol, 1eq, 76.3%ee) in 20mL THF at -8°C 0.46g iso-butylchloroformiate (3.38mmol, 1.1eq) and 0.8g diisopropylethylamine, Huenigs base (6.14mmol, 2eq) was added and stirred at this temperature for 1.5h. Afterwards 20mL DMSO and 0.64g potassium carbonate (4.61mmol, 1.5eq) were added, warmed to 20-25°C and stirred for 18h. To the mixture 15mL 1M NaOH solution was added and stirred for 3h. Afterwards pH 3 was achieved by adding 10% HCl. Subsequently 50ml MTBE was added, the organic layer was separated and washed with 30mL water and 30mL saturated NaCl solution. The organic layer was dried over sodium sulfate, filtered and the organic solvent was removed. After drying (<50mbar, 40°C) 0.9g of a brownish solid was isolated, containing 69.5area% racemic N-Boc-3-hydroxyadamant-1-yl glycine (62%, 1.92mmol, 3.8%ee).

### Example 6

### Alternative reaction procedure: Mesylchloride as activating reagent

To a solution of 1g (R)-N-Boc-3-hydroxyadamant-1-yl glycine (3.07mmol, leq, 76.3%ee) in 20mL THF at -8°C 0.40g mesyl chloride (3.38mmol, 1.1eq) and 0.8g Huenigs base (6.14mmol, 2eq) was added and stirred at this temperature for 1.5h. Afterwards 20mL DMSO and 0.64g potassium carbonate (4.61mmol, 1.5eq) was added, warmed to 20-25°C and stirred for 70h. To the mixture 15mL 1M NaOH solution was added and stirred for 3h. Afterwards pH 3 was achieved by adding 10% HCl. Subsequently 50ml MTBE was added, the organic layer was separated and washed with 30mL water and 30mL saturated NaCl solution. The organic layer was dried over sodium sulfate, filtered and the organic solvent was removed. After drying (<50mbar, 40°C) 0.65g of a brownish solid was isolated, containing 62.3area% racemic N-Boc-3-hydroxyadamant-1-yl glycine (40%, 1.92mmol, 5%ee).

### Example 7

The racemic N-Boc-3-hydroxyadamant-1-yl glycine obtained according to any of examples 1 to 6 was used as intermediate for the synthesis of Saxagliptin in the following sequence of reactions:

### 7.1 I Racemic resolution of N-Boc-3-hydroxyadamant-1-yl glycine

16.1g (1*R*,2*S*)-2-amino-1,2-diphenylethanol (75.5mmol, 0.7eq) were suspended in a 2L 3 neck round bottom flask equipped with a mechanic stirrer, a reflux condenser and a dropping funnel in 200mL ethyl acetate. 35g N-Boc-3-hydroxyadamant-1-yl glycine (107.6mmol, 1eq) dissolved in 600mL ethyl acetate were added slowly via the dropping funnel over a period of 5min. 100mL ethyl acetate were added via the dropping funnel to wash left over of N-Boc-3-hydroxyadamant-1-yl glycine. The mixture was stirred and after 20min a clear solution was observed. After 1h a white suspension occurs. The suspension was heated to 70°C and 400mL ethyl acetate were added slowly over a period of 2h at this temperature until the suspension was dissolved. The mixture was cooled slowly to 20-25°C over 14h under permanent stirring. The occurring precipitate was isolated by filtration leading to 53g of a white solid which was dried at 40°C/<40mbar. 24.7g of the N-Boc-3-hydroxyadamant-1-yl glycine amine salt was isolated with an enantiomeric excess of 86%. The solid was suspended in a 2L 3 neck round bottom flask equipped with a reflux condenser and a mechanic stirrer in 1000mL ethyl acetate. The suspension was heated to 70°C and 200mL ethyl acetate was added slowly over a period of 1.5h until a clear solution occurs. The mixture was slowly cooled to room temperature and stirred at that temperature for 2.5h while a white precipitate occurred. The precipitate was isolated via filtration (44g) and dried at 40°C/<40mbar leading to 20.8g of N-Boc-3-hydroxyadamant-1-yl glycine amine salt. The solid was dissolved in 200mL water and 100mL ethyl acetate. By adding 2M HCl pH 3 was adjusted. The organic phase was separated and the aqueous phase was extracted with 200mL ethyl acetate. The combined organic phase was dried over about 10g sodium sulfate. After filtration the organic solvent was removed via distillation and the residue was dried (40°C/<40mbar) leading to 13.9g N-Boc-3-hydroxyadamant-1-yl glycine (42.7mmol, 79%, 96.3%ee). From the combined mother liquors remaining N-Boc-3-hydroxyadamant-1-yl glycine can be isolated using the same procedure via acidification and extraction leading to N-Boc-3-hydroxyadamant-1-yl glycine mainly containing the R-isomer. The chiral amine was recovered by basification of the combined aqueous phases and extraction with ethyl acetate.

The *(R)*-N-Boc-3-hydroxyadamant-1-yl glycine which had not been reacted was again subsected to the process of the present invention.

### 7.2 Coupling of (S)-N-Boc-3-hydroxyadamant-1-yl glycine with (1S,3S,5S)-2-Azabicyclo[3.1.0]hexane-3-dcarboxamide hydrochloride

200mL ethyl acetate and 32mL *N*-methylmorpholine (286mmol, 5eq) were mixed in 500mL flask under nitrogen atmosphere and cooled to 0°C. Afterwards 19.0g (S)-N-Boc-3-hydroxyadamant-1-yl glycine (58.4mmol, 1eq) and 9.5g 1S,3S,5S)-2-Azabicyclo [3.1.0]hexane-3-dcarboxamide hydrochloride (58.4mmol, leq) were added and the mixture was stirred for 5min. 44.7g 50% ^{Ⓡ}T3P in butyl acetate (70mmol, 1.2eq) were slowly added at 0°C and the mixture was stirred at 0°C for 3h 30min. After IPC using HPLC that showed not complete conversion 7.5g 50% ^{Ⓡ}T3P in butyl acetate (11.7mmol. 0.2eq) was added and the mixture was stirred for 14h and warmed to 20-25°C. Afterwards the formed white solid was filtered off and the solid was washed two times each with 20mL ethyl acetate. The combined filtrate was hydrolyzed with 500mL 1M NaOH. The phases were separated and the aqueous phase was extracted two times each with 150mL ethyl acetate. The combined organic phases were washed with 200mL saturated NaCl solution. The aqueous phase was extracted with 150mL ethyl acetate and the combined organic phases were dried over about 15g Sodium sulfate. After filtration the organic solvent was removed via distillation (rotovapor, 200-40mbar, 40°C) and the residue was dissolved in about 200mL methylene chloride. The solvent was again removed (rotovapor, 600-40mbar, 40°C) and during this left over butyl acetate was distilled of azeotropically. After drying (<30mbar, 40°C) 20.1g [(1S)-2-[(1S,3S,5S)-3-(aminocarbonyl)-2-azabicyclo[3.1.0]hex-2-yl]-1-(3-hydroxy-tricyclo[3.3.1.13,7]dec-1-yl)-2-oxoethyl]-carbamic acid-1,1 -dimethylethyl ester (48.4mmol, 79%, purity: 83%) as a amorphous solid was isolated.

### 7.3 Dehydratisation of [(IS)-2-[(IS,3S,5S)-3-(aminocarbonyl)-2-azabicyclo[3.1.0] hex-2-yl]-1-(3-hydroxy-tricyclo[3.3.1.13,7]dec-1-yl)-2-oxoethyl]-carbamic acid-1,1-dimethylethyl ester

17.5g [(1S)-2-[(1S,3S,5S)-3-(aminocarbonyl)-2-azabicyclo[3.1.0] hex-2-yl]-1-(3-hydroxytricyclo[3.3.1.13,7]dec-1-yl)-2-oxoethyl]-carbamic acid- 1, 1 -dimethylethyl ester (40.4mmol, 1 eq) were dissolved in 270mL dry THF in a 1L 3-neck flask and cooled to 0°C. To the solution 16.4mL pyridine (201.8mmol, 5eq) was added followed by the slow addition of 11.3mL trifluoroacetic anhydride (80.8mmol, 2eq) via syringe. The now reddish solution was stirred at 0°C and the progress of the reaction was observed via HPLC. After 1h 30min complete conversion of the starting material was detected and 300mL 10% potassium carbonate solution was added followed by the addition of 150mL methanol leading to a mixture with pH >10. The reaction was stirred at 20-25°C until no intermediate was observed (IPC: HPLC). Afterwards the volume of the mixture was reduced about 150mL via distillation at the rotovapor. The remaining solution was extracted two times each with 350mL of a 1:1 mixture of diethyl ether and ethyl acetate. The organic phase was washed with 350mL 0.1M HCl solution and the aqueous phase was extracted with 75mL of 1:1 mixture of diethyl ether and ethyl acetate. The combined organic phases were washed with 200mL saturated sodium chloride solution and the aqueous phase was extracted with 75mL ethyl acetate. The combined organic phases were dried over about 15g sodium sulfate. Additional 10g charcoal was added and the suspension was stirred for 1.5h.After filtration the organic solvent was removed via distillation (600-40mbar/40°C) and 16.8g of a white amorphous solid were isolated. The solid was dried (<40mbar/40°C) and 15.0g (36.1mmol, 89%) of [(1S)-2-[(1S,3S,5S)-3-cyano-2-azabicyclo[3.1.0]hex-2-yl]-1-(3-hydroxytricyclo [3.3.1.13,7]dec-1-yl)-2-oxoethyl] carbamic acid-1,1-dimethylethyl ester as a white amorphous solid was isolated.

### 7.4 Synthesis of Saxagliptin via Deprotection

18.5g [(1S)-2-[(1S,3S,5S)-3-cyano-2-azabicyclo[3.1.0]hex-2-yl]-1-(3-hydroxytricyclo [3.3.1.13,7]dec-1-yl)-2-oxoethyl] carbamic acid-1,1-dimethylethyl ester (44.5mmol, 1eq) are dissolved in 180mL methylene chloride and 18.5mL methanol. The solution was cooled to 0°C and afterwards 18.5mL conc. hydrochloric acid (236mmol, 5.3eq) were added in 10min. after complete addition the mixture was stirred for 1h at 0°C than allowed to warm to 20°C and stirred for 4h 30min, stored at 4°C for 14h and again stirred at 20°C for 1h until complete conversion was detected (IPC: HPLC, starting material lower 1%). The mixture was hydrolyzed with 100mL Water (pH<1 followed by the addition of 100mL methylene chloride. The organic phase was separated and HPLC showed that it mainly contained the byproduct. The aqueous phase was adjusted to pH>11 by adding 5M sodium hydroxide solution. The mixture was extracted four times each with 80mL methylene chloride until no product could be detected in the aqueous phase (HPLC). The combined organic phases were dried over about 15g sodium sulfate. After filtration the organic solvent was removed via distillation (450mbar/30°C), dried under vacuum (<40mbar, 20°C) and 10.6g Saxagliptin was isolated containing small amounts of pyridine. The white solid was dissolved in 50mL toluene and the solvent was removed via distillation (<20mbar/30°) leading to 10.4g Saxagliptin (33.0mmol/74%) as white amorphous solid (purity HPLC: 93.7area%).

## Claims

1. Process for the preparation of racemic N-Boc-3-hydroxyadamant-1-yl glycine, wherein the educt (*R*)-N-Boc-3-hydroxyadamant-1-yl glycine or an isolated or not isolated derivative thereof is treated with a base under racemisation to yield racemic N-Boc-3-hydroxyadamant-1-yl glycine or to yield the racemic derivative thereof and hydrolysis of the racemic N-Boc-3-hydroxyadamant-1-yl glycine derivative to yield N-Boc-3-hydroxyadamant-1-yl glycine and optionally reacting or transforming the racemic N-Boc-3-hydroxyadamant-1-yl glycine in further process steps.

2. Process according to claim 1, wherein the educt (*R*)-N-Boc-3-hydroxyadamant-1-yl glycine is used in a purity of from 20 to 99.9%, based on the weight of the educt used.

3. Process according to at least one of the preceding claims, wherein the base is used in catalytic amounts, preferably in an amount of 0.1 to 0.5 wt.% based on the weight of the educt used.

4. Process according to at least one of claims 1 and 2, wherein the base is used in an essentially equivalent amount based on the educt used.

5. Process according to at least one of the preceding claims, wherein the racemisation is conducted at a pH of from 9 to 25.

6. Process according to at least one of the preceding claims, wherein the racemisation is conducted at a temperature of from -30 to 80°C.

7. Process according to at least one of the preceding claims, wherein the racemic N-Boc-3-hydroxyadamant-1-yl glycine obtained is subjected to a separation into its enantiomers.

8. Process according to at least one of the preceding claims, wherein (*R*)-N-Boc-3-hydroxyadamant-1-yl glycine is reacted with an activation reagent prior to racemisation.

9. Process according to claim 8 according to which the activation reagent is a carbodiimide, an acid chloride, a chloroformic acid ester, a fluorinated phenol, a sulfonic acid chloride or a carbonyl compound substituted by at least two heteroaromatic groups.

10. Process according to at least one of the preceding claims, wherein the racemisation is conducted in the presence of a non-protic polar solvent, preferably dimethylsulfoxide, acetonitrile, dimethylformamide, acetone, dimethylether, dioxane and mixtures thereof.

11. Process for the preparation of (S)-N-Boc-3-hydroxyadamant-1-yl glycine wherein the educt (*R*)-N-Boc-3-hydroxyadamant-1-yl glycine or an isolated or not isolated derivative thereof is treated with a base under racemisation to yield racemic N-Boc-3-hydroxyadamant-1-yl glycine or the racemic derivative thereof and hydrolysis of the racemic N-Boc-3-hydroxyadamant-1-yl glycine derivative to yield N-Boc-3-hydroxyadamant-1-yl glycine and removing *(R)*-N-Boc-3-hydroxyadamant-1-yl glycine and/or isolating *(S)*-N-Boc-3-hydroxyadamant-1-yl glycine.

12. Process for the preparation of Saxagliptin via *(S)*-N-Boc-3-hydroxyadamant-1-yl glycine, wherein the *(S)*-N-Boc-3-hydroxyadamant-1-yl glycine has been obtained according to claim 11.
